# EUROPEAN PATENT SPECIFICATION

(11) **EP 3 773 007 B1**
(45) Date of publication and mention of the grant of the patent: **24.06.2026**
(21) Application number: 19719910.2
(22) Date of filing: 04.04.2019
(51) Int. Cl.: A23L 27/30, C12N 9/10, A23L 33/21

(54) **PREBIOTIC COMPOSITIONS AND METHODS OF PRODUCTION THEREOF**
PRÄBIOTISCHE ZUBEREITUNGEN UND DEREN HERSTELLUNGSVERFAHREN
COMPOSITIONS PREBIOTIQUES ET LEURS METHODES DE PRODUCTION

(30) Priority: 04.04.2018 GB 201805578
(43) Date of publication of application: 17.02.2021
(73) Proprietor: Optibiotix Limited, Heslington, York YO10 5DG (GB)
(72) Inventor: O'HARA, Stephen Patrick, Heslington, York YO10 5DG (GB); HERNANDEZ, Oswaldo, Heslington, York YO10 5DG (GB); KOLIDA, Sofia, Heslington, York YO10 5DG (GB)
(74) Representative: Appleyard Lees IP LLP
(86) International application number: PCT/GB2019/050995
(87) International publication number: WO 2019/193358

(56) References cited:
- WO-A1-2013/026151
- WO-A1-2014/150127
- WO-A1-2017/214026
- WO-A1-2018/112189
- JP-A- H02 131 592
- JP-A- H04 288 093
- US-A1- 2002 065 245
- US-A1- 2003 236 399
- US-A1- 2012 214 752
- US-A1- 2015 305 380
- US-A1- 2017 181 443
- US-A1- 2017 208 847
- US-A1- 2017 303 565
- YU XUEJIAN ET AL: "High efficiency transformation of stevioside into a single mono-glycosylated product using a cyclodextrin glucanotransferase fromPaenibacillussp. CGMCC 5316", WORLD JOURNAL OF MICROBIOLOGY AND BIOTECHNOLOGY, SPRINGER NETHERLANDS, DORDRECHT, vol. 31, no. 12, 22 September 2015 (2015-09-22), pages 1983 - 1991, XP035575786, ISSN: 0959-3993, [retrieved on 20150922], DOI: 10.1007/S11274-015-1947-6

## Description

### Technical Field of the Invention

The invention relates to sweetened prebiotic compositions which have particular applications as functional food ingredients for foodstuffs and incorporation in meal replacement products or used by themselves to sweeten foods.

### Background to the Invention

The global sweetener market is currently dominated by sugar and is forecast to reach $112bn by 2022. There is an increasing move towards low calorie or calorie free sweeteners. A number of sweeteners, such as Steviol glycosides and Mogroside V are classified as high intensity sweeteners (HIS) and have reported sweet potencies relative to sucrose of approximately 150X and 400X respectively. However, a number of HIS are associated with bitter or off notes which reduce their appeal to consumers.

Prebiotics are substrates that are selectively utilized by host microorganisms, such as *lactobacilli* or *bifidobacteria,* conferring a health benefit, and are finding much increased application into the food sector. Prebiotics can be non-digestible food ingredients that are selectively metabolised by colonic bacteria which contribute to improved health. As such, their use can promote beneficial changes within the indigenous gut microbiota and they can help survivability of probiotics. They are distinct from most dietary fibres like pectin, celluloses, xylan, which have a global effect on gut bacterial populations and are not selectively metabolised in the gut. Criteria for classification as a prebiotic is that it must resist gastric acidity, hydrolysis by mammalian enzymes and absorption in the upper gastrointestinal tract, and it reach the colon in appropriate amount to be fermented by intestinal microbiota and selectively stimulate the growth and/or activity of intestinal bacteria associated with health and well-being.

The combination of enzymatically modified steviol glycosides with e.g., prebiotics, is disclosed in US 2017/303565 A1.

It is an object of the present invention to provide a prebiotic composition, or a composition comprising a prebiotic component, which can impart a sweet taste. In particular, it is an object of the present invention to provide a prebiotic composition, or a composition comprising a prebiotic component, which imparts a sweet taste, with low bitter and/or an undesirable after tastes. It is a further object of the present invention to provide a prebiotic composition, or a composition comprising a prebiotic component, which is not digested in the upper gastrointestinal tract of humans or animals and can therefore be used as calorie free, or substantially calorie free, functional ingredient which can improve gut microbiome diversity.

### Summary of the Invention

The present invention is defined in the appended claims, and relates to:
1) A prebiotic composition obtained by enzymatically modifying a high intensity sweetener glycoside by galactosylation and/or fructosylation in the presence of sucrose or lactose, and obtaining the enzymatically modified high intensity sweetener glycoside and a different oligosaccharide,
   wherein the prebiotic composition comprises:
   (i) the enzymatically modified high intensity sweetener glycoside; and
   (ii) the oligosaccharide.
2) A method for producing a sweetened prebiotic composition, the method comprising:
   contacting a high intensity sweetener glycoside with one or more enzymes effective to galactosylate and/or fructosylate the high intensity sweetener glycoside in the presence of sucrose and/or lactose so as to produce simultaneously different oligosaccharides; and
   optionally wherein the high intensity sweetener glycoside is selected from one or more of the following: Steviol glycosides or Mogroside, or derivatives thereof.
3) The use of the composition as defined above to provide a low calorie or calorie free sweet prebiotic.
4) The use of the composition as defined above, wherein the composition is incorporated, or is for incorporation, in or on, a foodstuff, a food supplement or a calorie restricted meal replacement product. Disclosed herein is a prebiotic composition comprising:
   (i) an enzymatically modified high intensity sweetener glycoside; and
   (ii) an oligosaccharide.

Further disclosed herein is a prebiotic composition comprising:
(i) an enzymatically modified high intensity sweetener glycoside; and
(ii) an enzymatically synthesised oligosaccharide.

Yet further disclosed herein is a synthetic prebiotic composition comprising:
(i) an enzymatically modified high intensity sweetener glycoside; and
(ii) an enzymatically synthesised oligosaccharide.

The term "synthetic" and "synthesised" are intended to mean products which are not naturally occurring or produced in nature. The terms do of course encompass products which are 'man made' using natural products, such as naturally derived precursor compositions and naturally derived enzymes.

The the high intensity sweetener glycoside may be enzymatically modified by galactosylation and/or fructosylation and/or deglycosylation.

The high intensity sweetener glycoside may be selected from one or more (or a combination) of the following: Steviol glycosides (such as Rebaudioside A) or Mogroside (such as Mogroside V), or derivatives thereof.

The enzyme or enzymes is or are microbially derived. The enzyme or enzymes may be derived from the *Aspergillus* genus. The enzymes may be derived from one or more of the following species of *Aspergillus*: *Aspergillus officinalis; Aspergillus aculeatus; Aspergillus awamori; Aspergillus carbonarius; Aspergillus cellulosae; Aspergillus oryzae; Aspergillus flavus; Aspergillus japonicas; Aspergillus nidulansl; or Aspergillus niger.*

The oligosaccharides obtained may be one the following: fructooligosaccharides (FOS), galactooligosaccharides (GOS), α-galactooligosaccharides, β-glucooligosaccharides, xylooligosaccharides and combinations thereof. It is preferred that the oligosaccharides obtained are one or more of the following: galactooligosaccharides (GOS) or fructooligosaccharides (FOS).

The composition may comprise up to about 5 % galactosylated and/or up to about 5 % fructosylated and/or 5 % deglycosylated high intensity sweetener glycoside. Preferably, the composition may comprise up to about 2 % galactosylated and/or up to about 2 % fructosylated and/or 2% deglycosylated high intensity sweetener glycoside. More preferably, the composition comprises up to about 1.5 % galactosylated and/or up to about 1.5 % fructosylated and/or 1.5% deglycosylated high intensity sweetener glycoside.

The high intensity sweetener glycoside may be galactosylated and/or fructosylated and/or deglycosylated during the oligosaccharide synthesis.

The high intensity sweetener glycoside will have been galactosylated and/or fructosylated and/or deglycosylated simultaneously with the synthesis of oligosaccharides.

In one disclosure, the high intensity sweetener glycoside comprises steviol glycosides which are modified by up to about 3 units of galactose or fructose by galactosylation and/or fructosylation.

In another disclosure, the high intensity sweetener glycoside comprises steviol glycosides which are modified by up to about 4 units of galactose or fructose by galactosylation and/or fructosylation.

In an alternative disclosure, the high intensity sweetener glycoside comprises steviol glycosides which are modified by about 4 or more units of galactose or fructose by galactosylation and/or fructosylation.

In one disclosure, the high intensity sweetener glycoside comprises mogrosides which are modified by up to about 3 units of galactose by galactosylation. In another disclosure, the high intensity sweetener glycoside comprises mogrosides which are modified by up to about 2 units of fructose by fructosylation. In an alternative disclosuret, the high intensity sweetener glycoside comprises mogrosides which are modified by about 2 or more units of fructose by fructosylation.

The steviosides may comprise a mixture of steviosides having different modifications. For example, the composition comprises a mixture of one or more of: (i) rebaudioside A, rebaudioside F, rebaudioside C, rubusoside or stevioside with 1 unit of fructose; (ii) stevioside with 2 units of fructose or rebaudioside A or rebaudioside C with 1 unit of fructose; and (iii) stevioside with 2 units of fructose or rebaudioside A or rebaudioside C with 1 unit of fructose. In the alternative, the composition comprises a mixture of one or more of: (i) rebaudioside A and rebaudioside C or stevioside with 1 unit of galactose; (ii) stevioside with 2 units of galactose or rebaudioside A or rebaudioside C with 1 unit of galactose; (iii) stevioside with 3 units of galactose or rebaudioside A or rebaudioside C with 2 units of galactose; and (iv) stevioside with 4 units of galactose or rebaudioside A or rebaudioside C with 2 units of galactose.

The mogrosides may comprise a mixture of mogrosides having different modifications. For example, the mogrosides may comprise a mixture of one or more of mogroside II, mogroside III, mogroside IV, mogroside V or mogroside VI. Alternatively, the mogrosides may comprise a mixture of one or more of: (i) mogroside V; (ii) mogroside IV, and (iii) mogroside III. Further alternatively, the mogrosides may comprise a mixture of one or more of: (i) mogroside III; (ii) mogroside IV; (iii) mogroside V; (iv) mogroside with 1 unit of fructose; and (v) mogroside with 2 units of fructose. Yet further, the mogrosides may comprises a mixture of: (i) mogroside IV; (ii) mogroside with 1 unit of galactose; (iii) mogroside V with 2 units of galactose; and (iv) mogroside V with 3 units of galactose.

All of the prebiotic compositions as described herein have been shown to advantageously form sweet natural and healthy fibres which are not digested in the human upper gastrointestinal tract and can therefore be used as calorie free, or substantially calorie free, functional ingredients. These sweet fibres have been developed as potential bulk sugar replacements as a product which has sweetness similar to sucrose but contain no, or substantially no, calories, whilst also advantageously improving microbiome diversity.

The components of the prebiotic compositions have been found to be significantly sweeter than all other samples, with the advantage of low off-flavours (e.g. bitterness, sourness, staleness, saltiness etc).

Disclosed herein is the use of the prebiotic compositions as herein above described as a low calorie or calorie free sweet prebiotic. It will be apparent to the skilled addressee that the composition may also be incorporated, or is for incorporation, in or on, a range of foodstuffs, food supplements or calorie restricted meal replacement products or used by itself to sweeten.

In certain disclosures, the composition may be in a powdered or granular form, and optionally, included in a sachet or jar so that a consumer can add the desired amount of the composition to a foodstuff.

The term "foodstuff" is intended to mean any material which can be safely ingested by a human or animal, including, but not limited to foods, beverages, cereals, bakery products, breaded and battered products, dairy products, confectionary, snacks, and meals. The term includes those products which require reconstitution prior to being cooked or eaten. The term also includes any food/nutritional supplements or medicaments (such as vitamin tablets or antibiotic liquids).

It will be apparent to the skilled addressee that the modified high intensity sweetener glycosides may be incorporated into a product, by way of blending or mixing the glycosides with other ingredients. Alternatively, the modified high intensity sweetener glycosides may be used to coat a product.

Further disclosed herein is a method for producing a sweetened prebiotic composition, the method comprising:
a) contacting high intensity sweetener glycosides with one or more enzymes effective to galactosylate and/or fructosylate the high intensity sweetener glycoside in the presence of different donors (such as sucrose and/or lactose), mainly disaccharides; and
b) obtaining the high intensity sweetener glycoside with different oligosaccharides during galactosylation and/or fructosylation of the high intensity sweetener glycoside so as to form sweetened prebiotic composition.

The high intensity sweetener glycoside in the method may be selected from one or more of the following: Steviol glycosides (such as Rebaudioside A), or Mogroside (such as Mogroside V), or derivatives thereof. Preferably, the high intensity sweetener glycoside is be selected from one or more of the following: Steviol glycosides, or Mogroside V, or derivatives thereof

The high intensity sweetener glycoside in the method may be galactosylated, and/or fructosylated, using one or more enzymes selected from: β-galactosidases and multi-enzyme complexes containing a wide range of carbohydrases, including arabanase, cellulase, β-glucanase, hemicellulose, pectinases and xylanase produced by a species/strain of *Aspergillus.*

The oligosaccharide in the method may be synthesized and be one or more of the following: fructooligosaccharides (FOS), galactooligosaccharides (GOS), β-gluco-oligosaccharides, α-galactooligosaccharides and xylooligosaccharides and combinations thereof. It is preferred that the synthesized oligosaccharide is selected from one or more of the following galactooligosaccharides (GOS) or fructooligosaccharides (FOS).

The sweetened prebiotic composition in the method may comprise up to about 5 % galactosylated and/or up to about 5 % fructosylated high intensity sweetener glycoside. Preferably, the composition in the method may comprise up to about 2 % galactosylated and/or up to about 2 % fructosylated high intensity sweetener glycoside. More preferably, the composition in the method comprises up to about 1.5 % galactosylated and/or up to about 1.5 % fructosylated high intensity sweetener glycoside.

The high intensity sweetener glycoside may be galactosylated and/or fructosylated in the presence of the enzyme and a disaccharide (donor).

The composition of the method may comprise a galactooligosaccharide or fructooligosaccharide.

The method may be employed to produce a composition as herein above described.

### Detailed Description of the Invention

The invention will now be described, by way of examples only.
Figure 1A shows the HPLC-DAD profile and the detection of Steviol glycosides described in Example 1, whereas Figure 1B shows the GC-FID profile and the detection of carbohydrates as described in Example 2;
Figure 2A shows the HPLC-DAD profile for the detection of Steviol glycosides as described in Example 1, whereas Figure 2B shows the GC-FID profile for detection of carbohydrates as described in Example 2;
Figure 3A shows the HPLC-DAD profile for the detection of Mogrosides as described in Example 3, whereas Figure 3B shows the GC-FID profile for the detection of carbohydrates as described in Example 3;

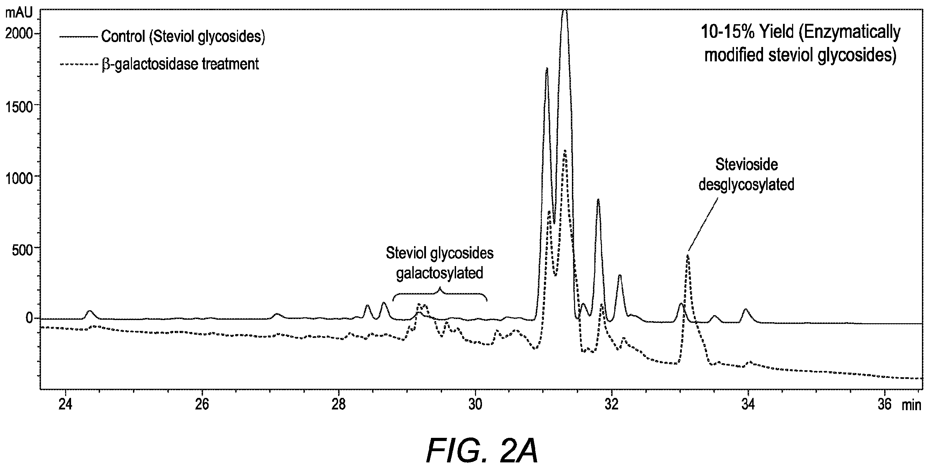

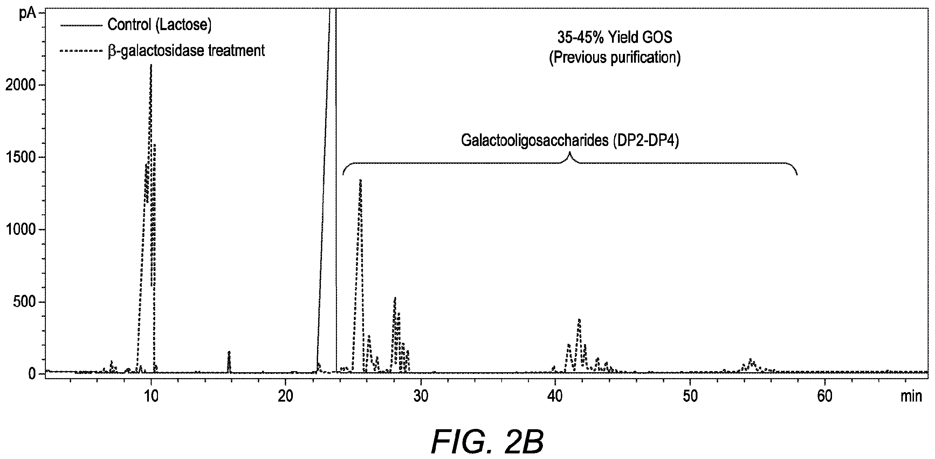

Figure 2A shows the HPLC-DAD profile for the detection of Steviol glycosides as described in Example 1, whereas Figure 2B shows the GC-FID profile for detection of carbohydrates as described in Example 2;

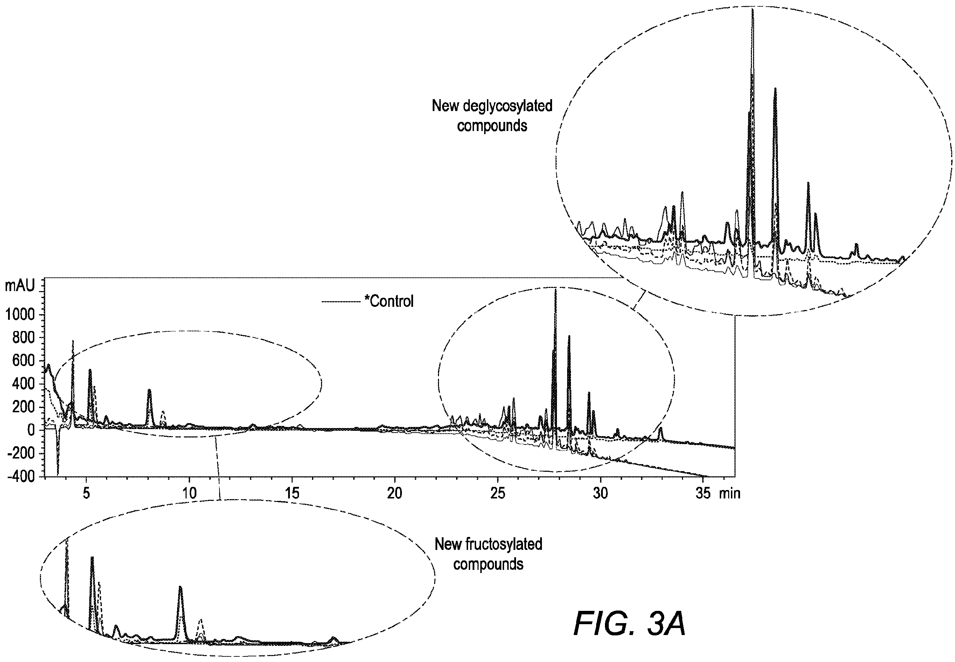

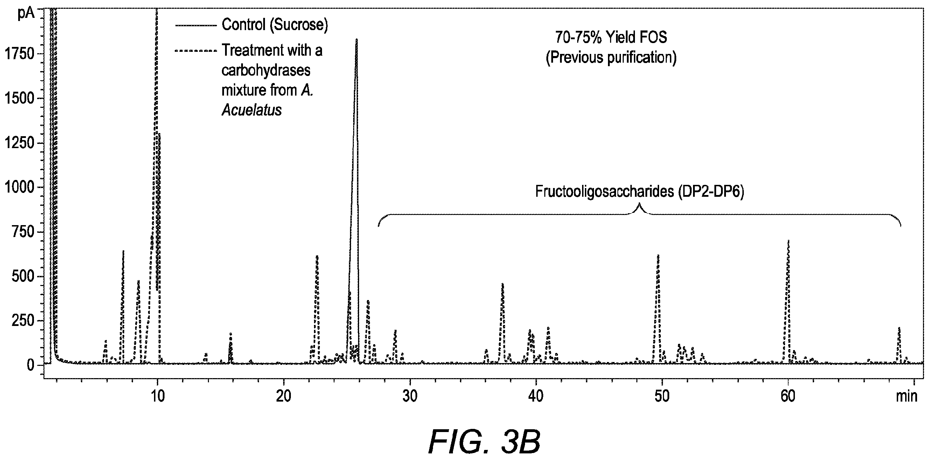

Figure 3A shows the HPLC-DAD profile for the detection of Mogrosides as described in Example 3, whereas Figure 3B shows the GC-FID profile for the detection of carbohydrates as described in Example 3;

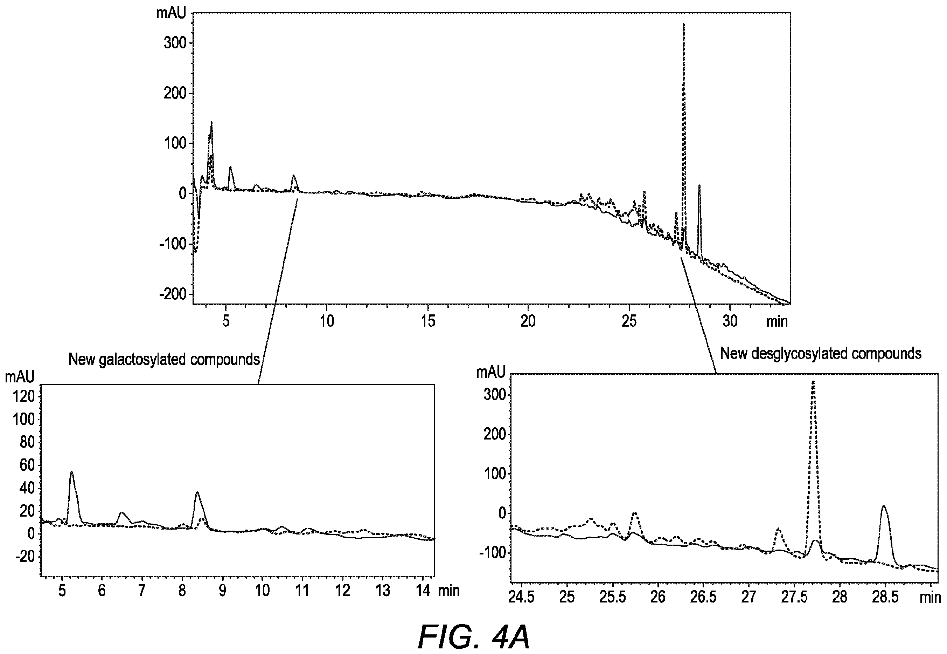

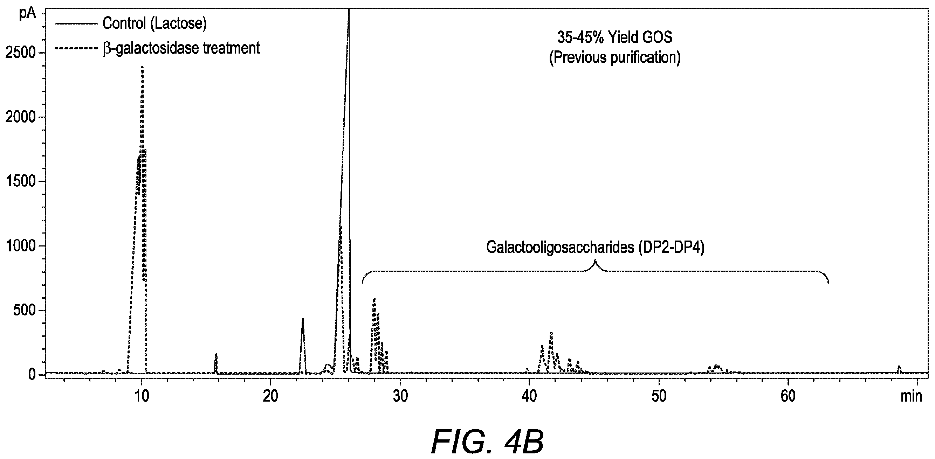

Figure 4A shows the HPLC-DAD profile for the detection of Mogrosides as described in Example 4, wherein Figure 4B shows the GC-FID profile for the detection of carbohydrates as described in Example 4;

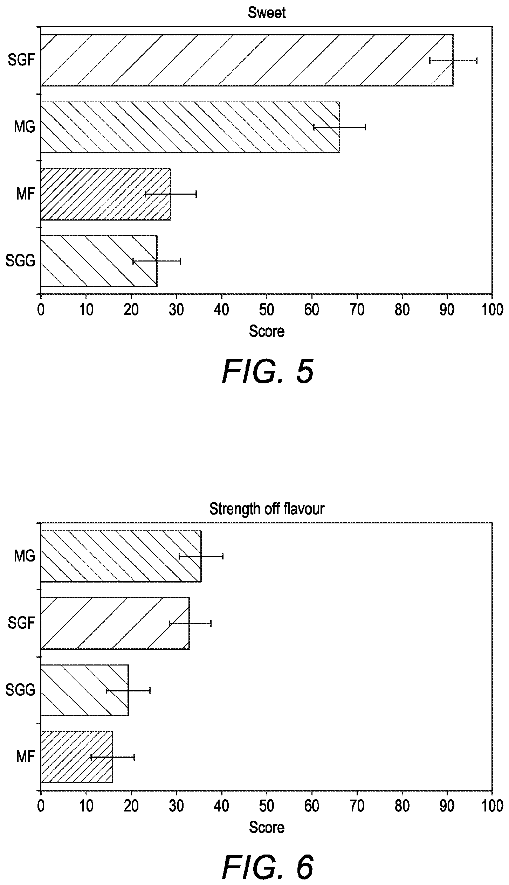

Figure 5 is a bar chart showing sensory test results for sweet taste (bars represent mean values, error bars extend +/- half LSD) for the samples tested in Example 5;
Figure 6 is a bar chart showing sensory test results for the strength of off flavours (bars represent mean values, error bars extend +/- half LSD) for the samples tested in Example 5;

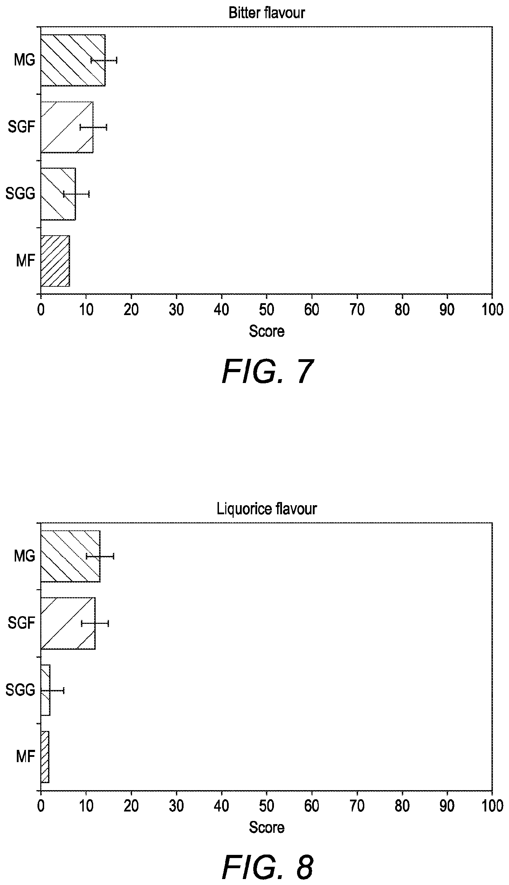

Figure 7 is a bar chart showing sensory test results for bitter taste (bars represent mean values, error bars extend +/- half LSD) for the samples tested in Example 5;
Figure 8 is a bar chart showing sensory test results for liquorice taste (bars represent mean values, error bars extend +/- half LSD) for the samples tested in Example 5;

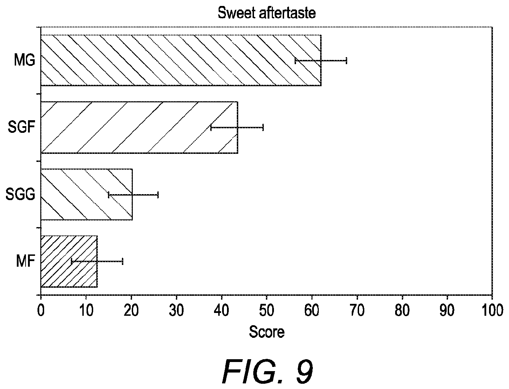

Figure 9 is a bar chart showing sensory test results for sweet aftertaste (bars represent mean values, error bars extend +/- half LSD) for the samples tested in Example 5;

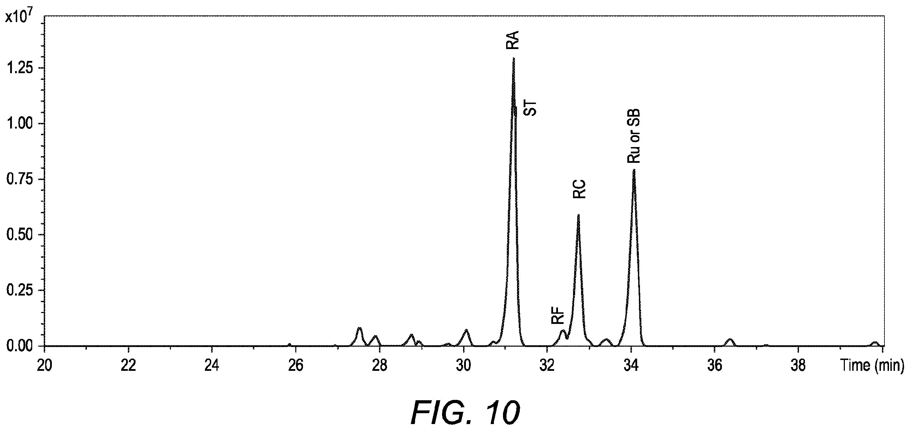

Figure 10 is a plot showing the HPLC-MS profile for enzymatically modified steviol glycosides with *A. acualetus* carbohydrases as described in Example 6. RA: rebaudioside A; ST: stevioside; RF: rebaudioside F; RC: rebaudioside C; Ru: Rubusoside; Sb: Steviolbioside;

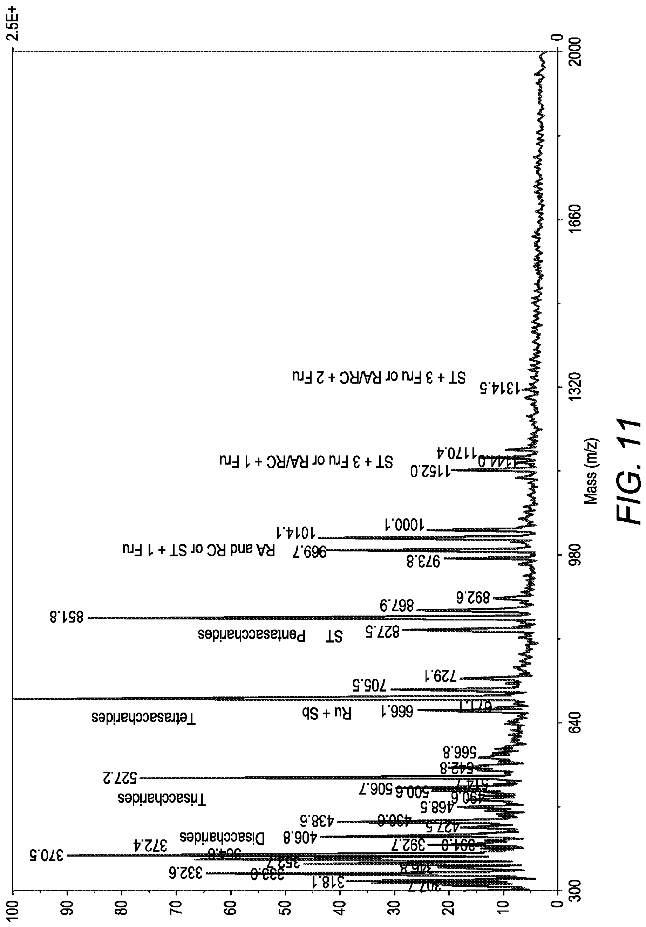

Figure 11 is a plot showing the MALDI-TOF profile for enzymatically modified steviol glycosides with *A. acualetus* carbohydrases as described in Example 6. RA: rebaudioside A; ST: stevioside; RF: rebaudioside F; RC: rebaudioside C; Ru: Rubusoside; Sb: Steviolbioside.; Fru: fructose;

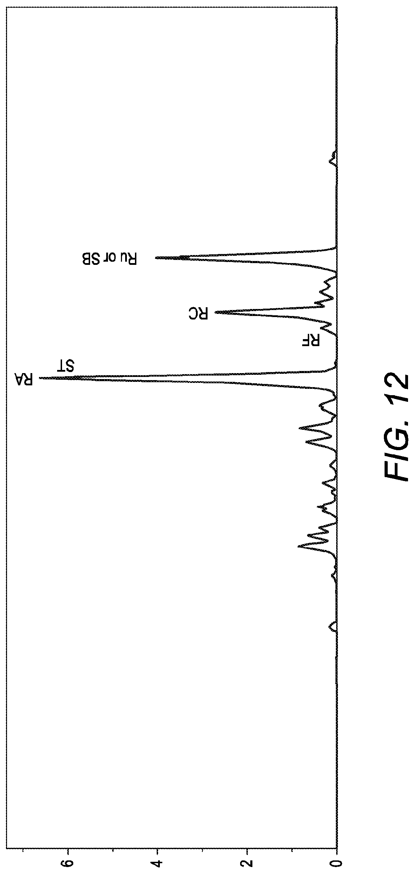

Figure 12 is a plot showing the HPLC-MS profile for enzymatically modified steviol glycosides with β-galactosidases as described in Example 6. RA: rebaudioside A; ST: stevioside; RF: rebaudioside F; RC: rebaudioside C; Ru: Rubusoside; Sb: Steviolbioside;

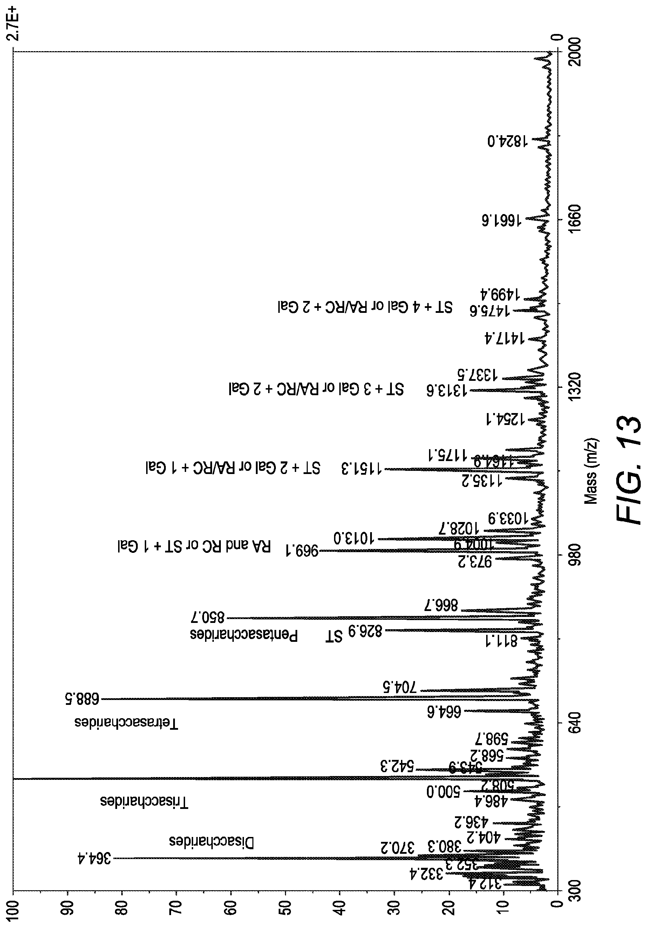

Figure 13 is a plot showing the MALDI-TOF profile for enzymatically modified steviol glycosides with β-galactosidases as described in Example 6. RA: rebaudioside A; ST: stevioside; RF: rebaudioside F; RC: rebaudioside C; Ru: Rubusoside; Sb: Steviolbioside.; Gal: galactose;

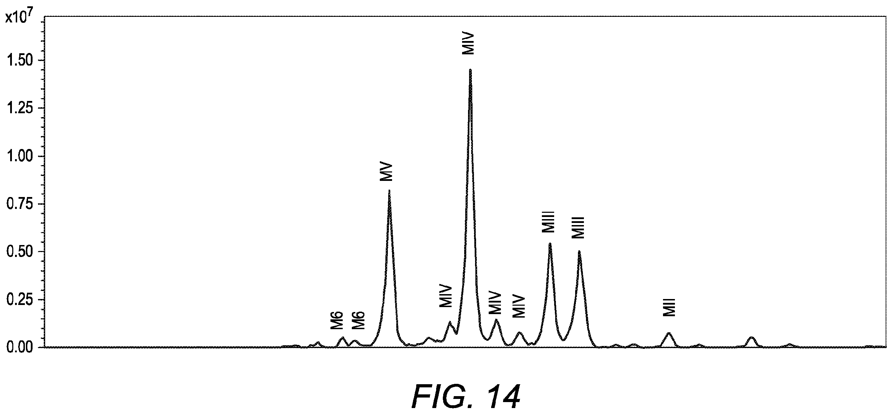

Figure 14 is a plot showing the HPLC-MS profile for enzymatically modified mogrosides with *A. acualetus* carbohydrases as described in Example 6. M: mogrosides.

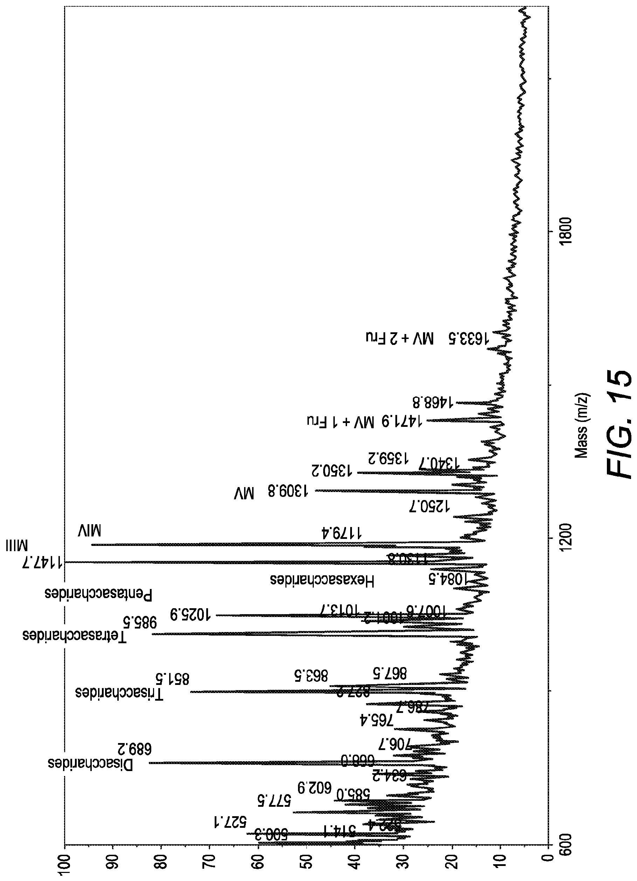

Figure 15 is a plot showing the MALDI-TOF profile for enzymatically modified mogrosides with *A. acualetus* carbohydrases. M: mogrosides; Fru: fructose;

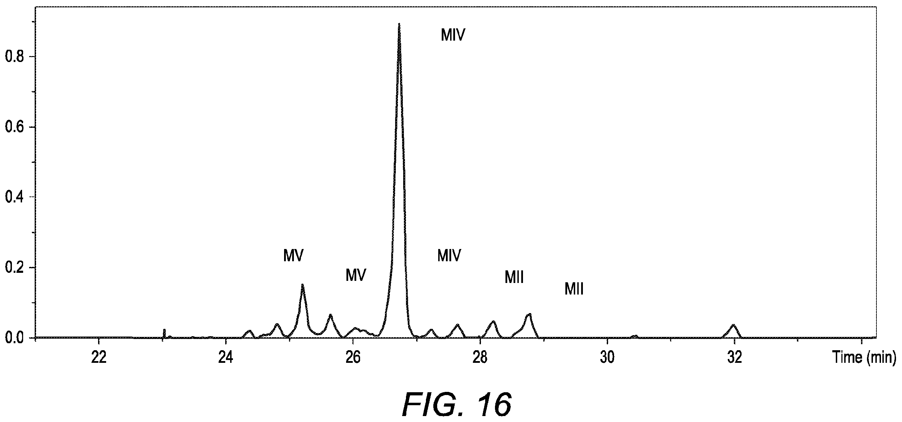

Figure 16 is a plot showing the HPLC-MS profile for enzymatically modified mogrosides with β-galactosidases as described in Example 6. M: mogrosides; and

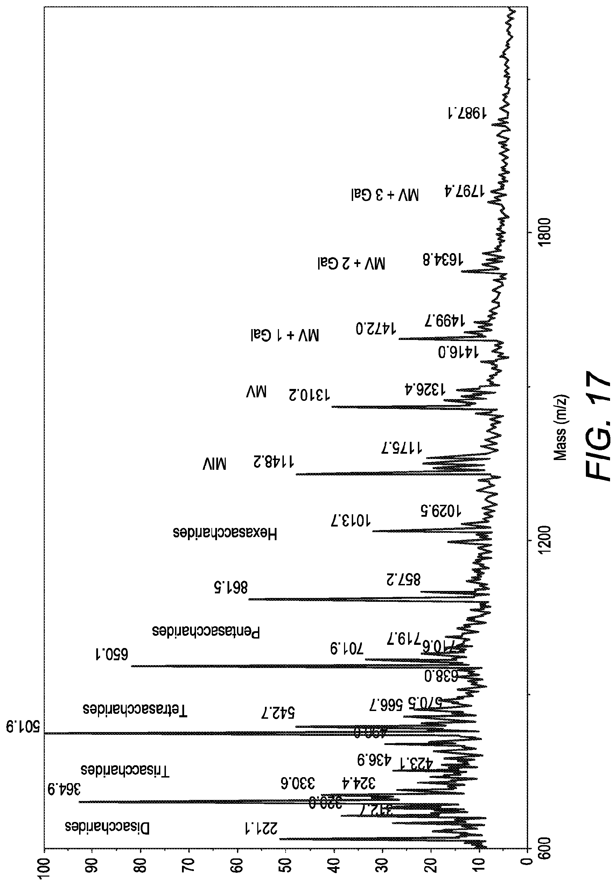

Figure 17 is a plot showing the MALDI-TOF profile for enzymatically modified mogrosides with β-galactosidases as described in Example 6. M: mogrosides; Gal: galactose.

The aim of these experiments was to determine the intensity of sweetness and any off flavours of a number of oligosaccharides and enzymatically modified high intensity glycosides, obtained during the same enzymatic reaction.

**Note:** examples where the high intensity glycoside sweetener is deglycosylated are provided for information, since not according to the present invention as claimed.

### Example 1 - Enzymatically modified steviol glycosides and FOS production

This experiment sought to investigate the potential yield and preferred enzymes for producing, fructosylated and/or deglycosylated steviol glycosides and FOS during the same enzymatic reaction. The enzymes investigated were carbohydrases complexes from *Aspergillus* and Inulinase from *Lactobacillus*(R&D)*.* The substrate was sucrose and steviol glycosides. The conditions used were 1.5% steviol glycosides, 60% sucrose, purification was by means of yeast fermentation and the drying process utilised lyophilisation and vacuum evaporation.

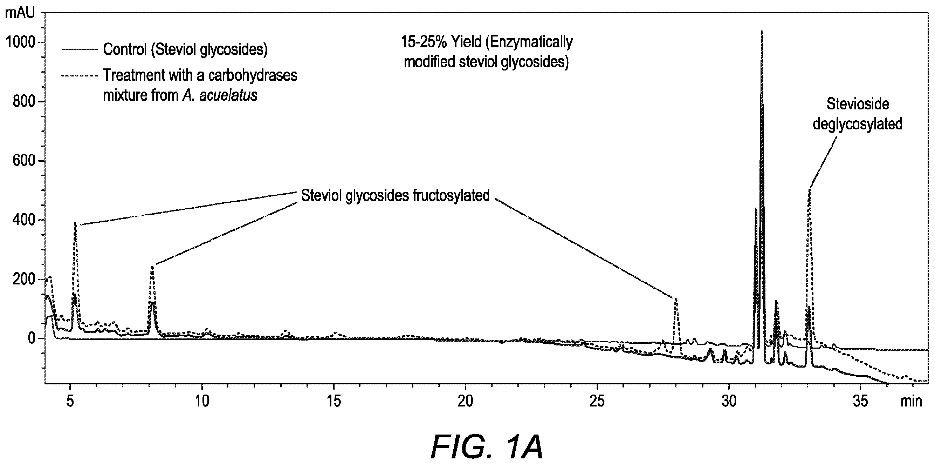

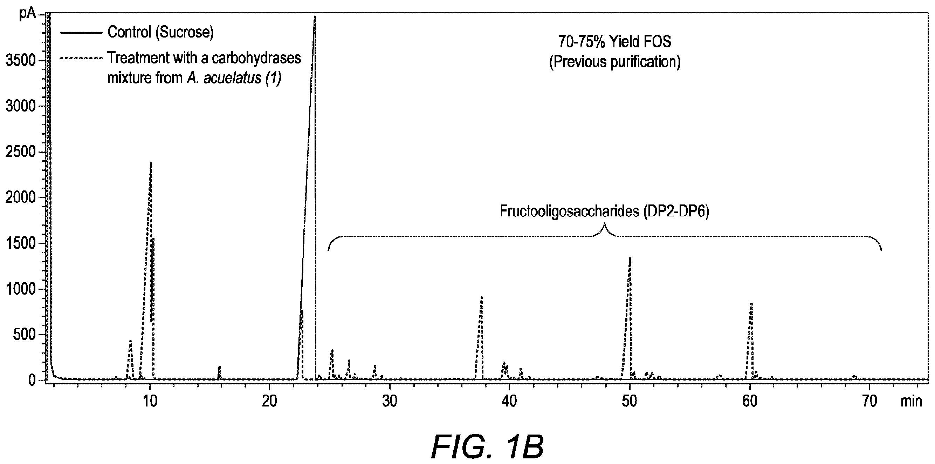

Figure 1A shows the HPLC-DAD profile and the detection of Steviol glycosides, whereas Figure 1B shows the GC-FID profile of the carbohydrates (trimethylsilyl oxime).

The best results were obtained utilising microbial enzymes complexes. The experiment suggested that the use of the commercial enzyme provided improved taste when the fructosylated and/or deglycosylated steviol glycosides was mixed with FOS generated during synthesis. The results therefore suggest that the mixture would be suitable for use as a prebiotic due to the high FOS concentration obtained during the enzymatic reaction.

### Example 2 - Enzymatically modified steviol glycosides and GOS production

This experiment sought to investigate the potential yield and preferred enzymes for producing galactosylated and /or deglycosylated steviol glycosides, and GOS during the same enzymatic reaction. The enzymes investigated were β-galactosidases from *Aspergillus* and *Bifidobacterium bifidum.* The substrates were lactose and steviol glycosides. The conditions were 1.5% steviol glycosides, 40% lactose. Purification was by means of yeast fermentation and the drying process was lyophilisation and rotovapor.

Figure 2A shows the HPLC-DAD profile for the detection of Steviol glycosides. Figure 2B shows the GC-FID profile for detection of carbohydrates (trimethylsilyl oxime).

The best results were obtained by using the β-galactosidases from *Aspergillus.* The results show the potential for the use of commercial enzymes to produce galactosylated and/or deglycosylated steviol glycosides and GOS obtained during the synthesis. The results therefore suggest that the high GOS concentration obtained during the enzymatic syntheis would be suitable for use as a prebiotic.

### Example 3 - Enzymatically modified mogrosides and FOS production

This experiment sought to investigate the potential yield and preferred enzymes for producing fructosylated and/or deglycosylated mogrosides, and FOS, during the same enzymatic reaction. The enzymes investigated were carbohydrases complexes from *Aspergillus acuelatus* and Inulinase from *Lactobacillus gasseri* (R&D). The substrate was sucrose and steviol glycosides. The conditions used were 1.5% steviol glycosides, 60% sucrose, purification was by means of yeast fermentation and the drying process utilised lyophilisation and vacuum evaporation.

Figure 3A shows the HPLC-DAD profile for the detection of Mogrosides. Figure 3B shows the GC-FID profile for the detection of carbohydrates (trimethylsilyl oxime).

The best results were obtained utilising microbial enzymes complexes. The experiment suggested that the use of the commercial enzyme provided improved taste when the fructosylated and/or deglycosylated mogrosides. It is believed that this is the first report of fructosylated mogrosides. The results therefore suggest that fructosylated and/or deglycosylated mogrosides and FOS, obtained simultaneously during the enzymatic reaction, would provide a good prebiotic mainly due to the high FOS concentration.

### Example 4 - Enzymatically modified mogrosides and GOS production

This experiment sought to investigate the potential yield and preferred enzymes for producing during the same enzymatic reaction, galactosylated and/or deglycosylated mogrosides and GOS. The enzymes investigated were β-galactosidases from *Aspergillus and Bifodabcaterium. bifidum.* The substrate used was lactose and mogrosides. The conditions used were 1.5% mogrosides, 40% lactose. Purification was performed using yeast fermentation and the drying process used lyophilisation and rotovapor.

Figure 4A shows the HPLC-DAD profile for the detection of Mogrosides. Figure 4B shows the GC-FID profile for the detection of carbohydrates.

The best results were obtained with were β-galactosidases from *Aspergillus.* It is believed that this is the first report of galactosylated mogrosides. The results therefore suggest that galactosylated and/or deglycosylated mogrosides, mixed with GOS, obtained simultaneously during the enzymatic reaction, would provide a good prebiotic due to the high GOS concentration.

### Example 5 - Sensory Test Data of Modified HIS

A trained sensory panel at the Reading Sensory Science Centre (UK) were employed for sensory profiling of the samples. There were 10 panellists with between 1 and 9 years' experience. A QDA (quantitative descriptive analysis) profiling approach was taken. The panel used the same vocabulary that they had developed as a consensus for the tasting sessions including the term liquorice flavour which is characteristic note of steviol glycosides. The panel were retrained at the start of the sample set over 3 separate tasting sessions. This re-training focused on ensuring that they could reliably score sweetness relative to the new concentration of sucrose standard positions.

Rating was carried out independently using unstructured lines scales (scaled 0-100), in duplicate, in isolated sensory booths. However, in order to improve discrimination for sweetness, the four sucrose samples were used as standards and the mean values for each of these samples, as agreed by the panel, are shown in Table 1 below.

**Table 1**

| Standard Number | Sucrose Concentration (% w/v) | Mean Rating (0-100) |
|---|---|---|
| 1 | 2.0 | 10 |
| 2 | 4.0 | 35 |
| 3 | 6.0 | 75 |
| 4 | 8.0 | 100 |

At the start of each scoring session the panel tasted the four reference samples in order of increasing strength to re-familiarise themselves with the positioning of these levels of sweetness on the line scale. The reference samples (10 mL) were served in transparent polystyrene cups (30 mL). They then palate cleansed with warm filtered tap water and low salt crackers (Carr's water crackers) before commencing the sample tasting session, and again between each sample scoring session.

Samples, labelled with random 3 digit codes, were presented in a balanced presentation order in a monadic sequential manner with a maximum of 6 samples per day. Samples were served at 23-24°C (room temperature) with air conditioning of the room set to 23 °C.

The panel used 15 attributes to define samples, as shown in Table 2 below, where mean scores (0-100) for mogrosides (M) and steviol glycosides (SG) modified using two different glycosidase mixtures (F: Example 1 and 3 and G: Example 2 and 4) as shown.

**Table 2**

| | **MF** | | **MG** | | **SGF** | | **SGG** | | **Fisher's LSD Value** | **Sample Significant (p)** |
|---|---|---|---|---|---|---|---|---|---|---|
| Sweet Taste | 28,6 | ^{c} | 65,8 | ^{b} | 91,1 | ^{a} | 25,5 | ^{c} | 10,9 | <.0001 |
| Overall Off Flavour | 15,5 | ^{b} | 35,3 | ^{a} | 33,1 | ^{a} | 19,1 | ^{b} | 9,3 | 0,0005 |
| Bitter Taste | 7,8 | ^{bc} | 14,1 | ^{a} | 11,9 | ^{ab} | 6,6 | ^{bc} | 5,8 | 0,0027 |
| Liquorice Flavour | 2,1 | ^{b} | 13,2 | ^{a} | 12,0 | ^{a} | 1,8 | ^{b} | 5,8 | <.0001 |
| Cardboard(Stale) | 4,0 | ^{ab} | 5,4 | ^{a} | 0,6 | ^{c} | 1,0 | ^{c} | 2,9 | 0,0145 |
| CandyFloss | 1,1 | | 5,1 | | 6,6 | | 2,6 | | 5,5 | 0,2289 |
| Sour/RancidDairy | 1,0 | | 2,4 | | 2,2 | | 4,2 | | 3,6 | 0,6943 |
| Metallic Taste | 5,9 | | 5,4 | | 4,3 | | 3,2 | | 4,7 | 0,5841 |
| Salty Taste | 1,9 | | 2,1 | | 2,7 | | 3,7 | | 3,5 | 0,8737 |
| CrustyBread Flavour | 1,1 | | 0,4 | | 0,7 | | 0,4 | | 1,5 | 0,7387 |
| Perfume | 0,5 | | 0,0 | | 0,0 | | 0,0 | | 0,6 | 0,4434 |
| Sweet Aftertaste | 20,2 | ^{c} | 43,2 | ^{b} | 61,8 | ^{a} | 12,0 | ^{c} | 11,4 | <.0001 |
| Bitter Aftertaste | 8,4 | | 9,0 | | 8,7 | | 6,0 | | 4,8 | 0,1170 |
| Liquorice Aftereffect | 2,2 | ^{b} | 8,3 | ^{a} | 8,2 | ^{a} | 1,5 | ^{b} | 4,5 | 0,0009 |
| Cooling Aftereffect | 0,1 | | 2,1 | | 0,6 | | 0,2 | | 1,8 | 0,2241 |

Figures 5 to 9 illustrate the sensory test data for the important sweet, off flavor, bitter, liquorice, or sweet aftertaste.

Table 3 below shows the equivalent sucrose and relative sweetness values of the samples tested.

**Table 3**

| **Sample** | **Equivalent Sucrose (%) to x% saccharide** | | | **Relative Sweetness (sucrose = 100) (RS)** | | |
|---|---|---|---|---|---|---|
| | **Mean** | **Min** | **Max** | **Mean** | **Min** | **Max** |
| MF | 0,9 | 0,4 | 1,4 | 18 | 8 | 28 |
| MG | 1,8 | 1,3 | 2,3 | 36 | 25 | 46 |
| SGF | 2,4 | 2,2 | 2,6 | 48 | 43 | 52 |
| SGG | 0,8 | 0,5 | 1,2 | 17 | 10 | 24 |

### Example 6 - Impact of MV-FOS, MV-GOS, SG-FOS and SG-GOS on the human gut microbiome

Experiments were conducted to assess the impact of MV-FOS, MV-GOS, SG-FOS and SG-GOS (1% w/v) on the metabolic activity of the human gut microbiome.

Matrix-assisted laser desorption ionization time-of-flight (MALDI-TOF) spectra were recorded by using a Voyager DE-PRO mass spectrometer (Applied Biosystems) equipped with a nitrogen laser emitting at 337 nm with a 3 ns, and 3 Hz frequency. Ions generated by laser desorption were introduced into a time of flight analyzer (1.3 m flight path) with an acceleration voltage of 25 kV, 94% grid voltage, 0.075% ion guide wire voltage, and a delay time of 400 ns in the linear positive ion mode. Mass spectra were obtained over the m/z range 100-5000. 2,5-Dihydroxybenzoic acid (>98%, Fluka) at a concentration of 10 mg/ml in water (Milli-Q water, Millipore, Bedfor, USA) was used as matrix. Samples were diluted 1:100 in water and then, mixed with the matrix solution at a ratio of approximately 1:3. 1 µL of this solution was spotted onto a flat stainless-steel sample plate and dried in air. External mass calibration was applied using the monoisotopic [M + H]+ values of of des-Arg1 Bradykinin, Angiotensin I, Glu1-Fibrino-peptide B, ACTH (1-17), ACTH (18-39), ACTH (7-38) and Insuline (Bovine). of the Calibration Mixtures 1 and 2, Sequazyme Peptide Mass Standards Kits; Applied Biosystems.

Separation and analysis of enzymatically modified steviol glycosides and mogrosides by LC-MS was performed at 25°C on a C18 column (150 mm x 2.1 mm, 3.5 mm particle size, ThermoFisher) at a flow rate of 0.1 mL/min with a solvent gradient of acetonitrile and water (0.1% formic acid). All experiments were carried out on a Finnigan Surveyor pump with quaternary gradient system coupled to a Finnigan LCQ Deca ion trap mass spectrometer using an ESI interface. Sample injections (10 mL) were carried out by a Finnigan Surveyor autosampler. All instruments (Thermo Fisher Scientific, San Jose, CA, USA), and data acquisition were managed by Xcalibur software (1.2 version; Thermo Fisher Scientific).

The impact of MV-FOS, MV-GOS, SG-FOS and SG-GOS (1% w/v) on the metabolic activity of the human gut microbiome was investigated in pH and temperature-controlled batch cultures. Impact on the concentration of organic acids was compared to short chain fructooligosaccharides (prebiotics positive control; FUJIFILM Wako Chemicals, Germany), and a carbohydrate negative control. Fructooligosaccharides and galactooligosaccharides produced by the activity the same enzymes used for the synthesis of modified MV and SG (1% w/v) were also tested as well as non-modified MV and SG (0.2% w/v).

Freshly voided faecal samples were obtained from five healthy adults, free from gastrointestinal disorders who had not taken antibiotics for 6 months prior to the study and prebiotics and/or probiotics for 6 weeks prior to the study.

Sterile fermenters (20 mL working volume, Soham scientific, Ely, UK) were filled with pre-reduced sterile basal media consisting of: peptone water (Oxoid, Basingstoke, UK) 2 g L⁻¹; yeast extract (Oxoid, Basingstoke, UK) 2 g L⁻¹; NaCl 0.1 g L⁻¹; K₂HPO₄ 0.04 g L⁻¹; KH₂PO₄ 0.04 g L⁻¹; MgSO₄.7H₂O 0.01 g L⁻¹; CaCl₂.6H₂O 0.01 g L⁻¹; NaHCO₃ 2 g L⁻¹; haemin 0.05 g L⁻¹; cysteine.HCl 0.5 g L⁻¹; bile salts 0.5 g L⁻¹, vitamin K1 10 µL; Tween 80 2 mL (Sigma Aldrich) and sparged with oxygen-free N₂ to establish and maintain anaerobic conditions. Stirring was achieved using magnetic stirrers. Test carbohydrates (1%w/v) were added in designated vessels just prior to inoculation with the faecal slurry from a single donor (10 % v/v prepared in anaerobic phosphate buffered saline). All tests for a single donor were carried out in parallel. Fermentation temperature was maintained at 37 °C by means of a circulating water bath. Automated pH controllers (Fermac 260; Electrolab UK) kept culture pH within a range of 6.7 and 6.9 by adding 0.5 M NaOH and 0.5 M HCl as required. Fermentations were run for a period of 24 h and samples were withdrawn at 0, 5, 10, and 24 h for organic acid analysis. Table 4 below shows the results of the fermentation runs.

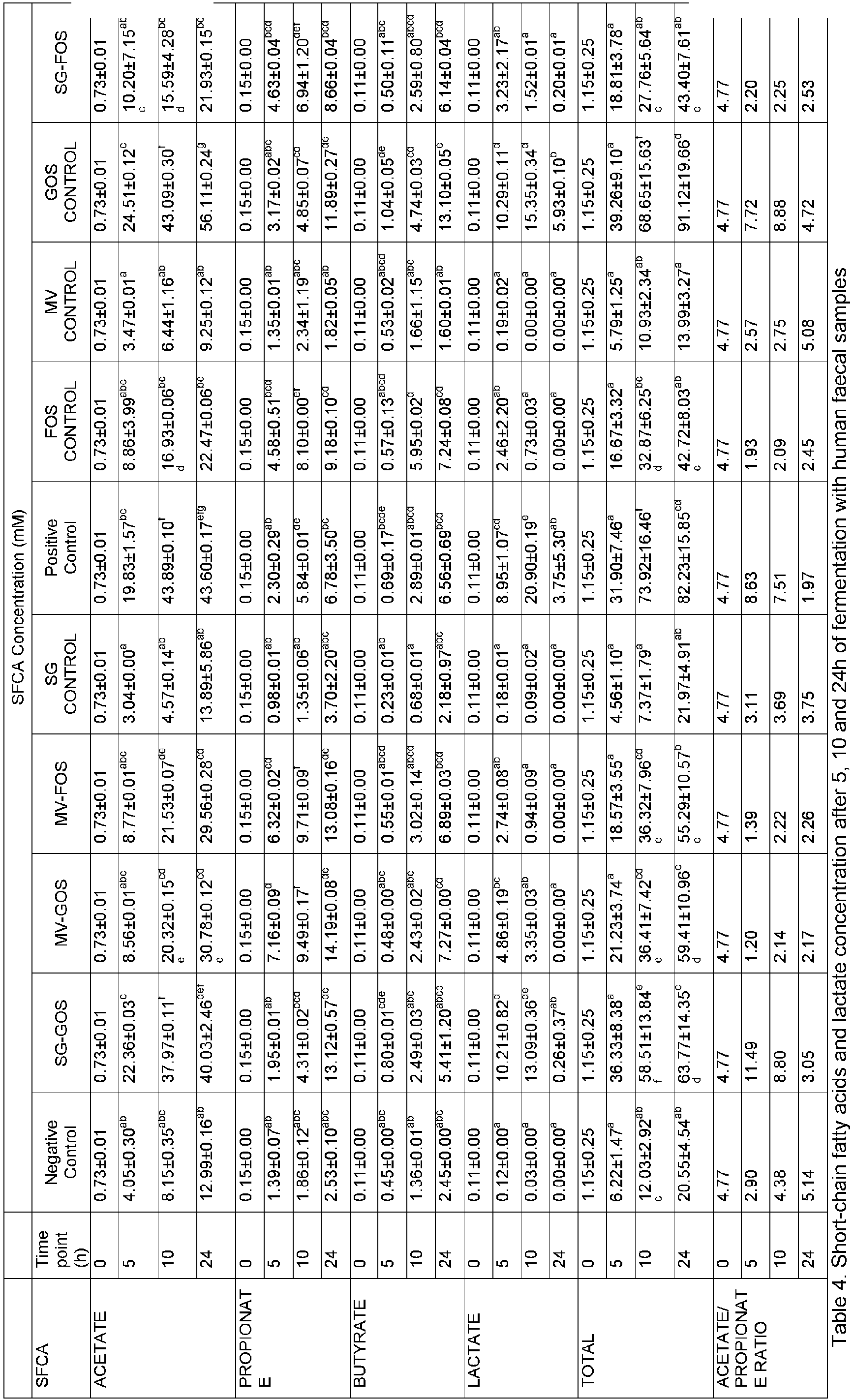

Organic acid (OA) concentrations were determined by gas chromatography equipped with flame ionisation detector (GC-FID) based on the method described by Richardson et al (1989) using 2-ethyl butyric acid as an internal standard. A gas chromatograph analyser (Agilent/HP 6890) equipped with a Flame Ionization Detector (FID) and an HP-5MS column (30 m × 0.25 mm) with a 0.25 µm coating (Crosslinked (5%-Phenyl)-methylpolysiloxane, Hewlett Packard, UK) was used for SCFA measurements. Helium was used as carrier gas at a flow rate of 1.7 mL/min (head pressure 133 KPa). Oven initial temperature was set at 63 °C, followed by a temperature ramp of 15 °C/min to 190 °C and held constant for 3 minutes. A split ratio of 100:1 was used. The appearance of OA in the chromatograms was confirmed based on the retention times of the respective commercial OA standards (Lactic acid, Acetic acid, Propionic acid and Butyric acid) (Sigma-Aldrich, UK)

With reference to Figures 10 to 17, in general, SG-GOS and SG-FOS showed and modification of the steviol glycosides by the desglycosilation and galactosylation and fructosylation, respectively up to 3 to 4 more units of lactose or fructose. This behaviour was also found for MV-GOS and MV-FOS, in this case the the mogrosides were up to galacosylated by 3 galactose units and fructosylated by 2 fructose units.

SG-GOS was fermented rapidly as indicated by significant increases in the levels of lactate at 5 and 10h of fermentation, behaving in a similar manner to the prebiotic control and GOS. Lactate is a fermentation intermediate, that is rapidly utilised through cross-feeding by other members of the gut microbiome. Lactate accumulates in culture when the rate of production is higher compared to the rate of utilisation and it is characteristic of rapid gut microbiome fermentation rates observed during the saccharolysis of oligosaccharides. Acetate, propionate and butyrate concentrations were also significantly higher compared to the negative control and followed similar patterns to those observed by the prebiotic control and GOS.

10h of fermentation, also observed with the positive control and levels were significantly lower compared to the prebiotic control but very similar to FOS, indicating slower fermentation rates. Acetate, propionate and butyrate concentrations were all significantly higher compared to the negative control and very similar to those in the FOS fermentations but significantly lower to the prebiotic control in terms of acetate formation. In the MV-GOS cultures lactate accumulation was significantly lower compared to GOS and the prebiotic control, indicating less rapid fermentation. Acetate concentrations were significantly higher compared to the negative control and gradually increased over the fermentation period, following similar patterns to the prebiotic control and GOS, albeit at lower levels. MV-GOS significantly increased propionate concentrations, with levels being significantly higher compared to the prebiotic control and GOS. Significant increases in butyrate were observed after 24h fermentation and were comparable to those with the prebiotic control and GOS.

MV-FOS metabolite formation followed identical patterns to MV-GOS with the exception of butyrate which did not increase significantly.

Overall, the fermentation behaviour of the compounds synthesised shows very close similarities to that of commercially available prebiotics. Their impact on the metabolic activity of the human gut microbiome is characteristic of oligosaccharide saccharolysis. They all increased significantly acetate but also propionate and butyrate, organic acids with important role in cholesterolgenesis, appetite regulation, tight junction integrity and immunomodulation.

## Claims

1. A prebiotic composition obtained by enzymatically modifying a high intensity sweetener glycoside by galactosylation and/or fructosylation in the presence of sucrose or lactose, and obtaining the enzymatically modified high intensity sweetener glycoside and a different oligosaccharide, wherein the prebiotic composition comprises:
(i) the enzymatically modified high intensity sweetener glycoside; and
(ii) the oligosaccharide.

2. The composition as claimed in claim 1, wherein the high intensity sweetener glycoside is selected from one or more of the following: Steviol glycosides or Mogroside, or derivatives thereof.

3. The composition as claimed in claim 2, wherein the Steviol glycoside comprises Rebaudioside A or the Mogroside comprises Mogroside V.

4. The composition as claimed in claim 1, wherein the oligosaccharide is one or more of the following: galactooligosaccharides (GOS) or fructooligosaccharides (FOS).

5. The composition as claimed in any preceding claim, wherein the composition comprises up to about 5% galactosylated and/or up to about 5 % fructosylated high intensity sweetener glycoside; or
wherein the composition comprises up to about 2% galactosylated and/or up to about 2% fructosylated high intensity sweetener glycoside; or
wherein the composition comprises up to about 1.5% galactosylated and/or up to about 1.5% fructosylated high intensity sweetener glycoside.

6. The composition as claimed in any preceding claim, wherein the high intensity sweetener glycoside has been galactosylated and/or fructosylated simultaneously with the synthesis of oligosaccharides.

7. The composition as claimed in claim 6, wherein the high intensity sweetener glycoside comprises steviol glycosides which are modified by up to about 3 units of galactose or fructose by galactosylation and/or fructosylation; or
wherein the high intensity sweetener glycoside comprises steviol glycosides which are modified by up to about 4 units of galactose or fructose by galactosylation and/or fructosylation; or.
wherein the high intensity sweetener glycoside comprises steviol glycosides which are modified by more than 4 units of galactose or fructose by galactosylation and/or fructosylation.

8. The composition as claimed in claim 6, wherein the high intensity sweetener glycoside comprises mogrosides which are modified by up to about 3 units of galactose by galactosylation; or
wherein the high intensity sweetener glycoside comprises mogrosides which are modified by up to about 2 units of fructose by fructosylation.

9. The composition as claimed in any one of claims 3 to 7, wherein the steviosides comprise a mixture of steviosides having different modifications; and optionally wherein the composition comprises a mixture of one or more of: (i) rebaudioside A, rebaudioside F, rebaudioside C, rubusoside or stevioside with 1 unit of fructose; (ii) stevioside with 2 units of fructose or rebaudioside A or rebaudioside C with 1 unit of fructose; and (iii) stevioside with 2 units of fructose or rebaudioside A or rebaudioside C with 1 unit of fructose; or
wherein the composition comprises a mixture of one or more of: (i) rebaudioside A and rebaudioside C or stevioside with 1 unit of galactose; (ii) stevioside with 2 units of galactose or rebaudioside A or rebaudioside C with 1 unit of galactose; (iii) stevioside with 3 units of galactose or rebaudioside A or rebaudioside C with 2 units of galactose; and (iv) stevioside with 4 units of galactose or rebaudioside A or rebaudioside C with 2 units of galactose.

10. The composition as claimed in any one of claims 3 to 6 and 8, wherein the mogrosides comprise a mixture of mogrosides having different modifications; and optionally wherein the mogrosides comprise a mixture of one or more of mogroside II, mogroside III, mogroside IV, mogroside V or mogroside VI; or
wherein the mogrosides comprise a mixture of one or more of: (i) mogroside V; (ii) mogroside IV, and (iii) mogroside III; or
wherein the mogrosides comprise a mixture of one or more of: (i) mogroside III; (ii) mogroside IV; (iii) mogroside V; (iv) mogroside with 1 unit of fructose; and (v) mogroside with 2 units of fructose; or
wherein the mogrosides comprise a mixture of: (i) mogroside IV; (ii) mogroside with 1 unit of galactose; (iii) mogroside V with 2 units of galactose; and (iv) mogroside V with 3 units of galactose.

11. Use of the composition as claimed in any preceding claim to provide a low calorie or calorie free sweet prebiotic.

12. Use of the composition as claimed in any one of claims 1 to 10, wherein the composition is incorporated, or is for incorporation, in or on, a foodstuff, a food supplement or a calorie restricted meal replacement product; and optionally wherein the composition is in a granular form, and optionally, included in a sachet or jar.

13. A method for producing a sweetened prebiotic composition, the method comprising:
contacting a high intensity sweetener glycoside with one or more enzymes effective to galactosylate and/or fructosylate the high intensity sweetener glycoside in the presence of sucrose and/or lactose so as to produce simultaneously different oligosaccharides; and optionally wherein the high intensity sweetener glycoside is selected from one or more of the following: Steviol glycosides or Mogroside, or derivatives thereof.

14. The method of claim 13, wherein the high intensity sweetener glycoside is galactosylated and/or fructosylated using one or more enzymes selected from: carbohydrase mixtures obtained from *Aspergillus* sp. and β-galactosidases; and optionally wherein the oligosaccharide synthesized is one or more of the following: galactooligosaccharides (GOS) or fructooligosaccharides (FOS).

15. The method as claimed in any one of claims 13 or 14, wherein the sweetened prebiotic composition comprises up to about 5 % galactosylated and/or up to about 5 % fructosylated high intensity sweetener glycoside.

## Patentansprüche

1. Präbiotische Zusammensetzung, erhalten durch enzymatisches Modifizieren eines Süßungsglycosids hoher Intensität durch Galactosylierung und/oder Fructosylierung in Gegenwart von Saccharose oder Lactose und Erhalten des enzymatisch modifizierten Süßungsglycosids hoher Intensität und eines anderen Oligosaccharids, wobei die präbiotische Zusammensetzung umfasst:
(i) das enzymatisch modifizierte Süßungsglycosid hoher Intensität; und
(ii) das Oligosaccharid.

2. Zusammensetzung nach Anspruch 1, wobei das Süßungsglycosid hoher Intensität ausgewählt ist aus einem oder mehreren der Folgenden: Steviolglycosiden oder Mogrosid oder Derivaten davon.

3. Zusammensetzung nach Anspruch 2, wobei das Steviolglycosid Rebaudiosid A umfasst oder das Mogrosid Mogrosid V umfasst.

4. Zusammensetzung nach Anspruch 1, wobei das Oligosaccharid eines oder mehrere der Folgenden ist: Galactooligosaccharide (GOS) oder Fructooligosaccharide (FOS).

5. Zusammensetzung nach einem der vorstehenden Ansprüche, wobei die Zusammensetzung bis zu etwa 5 % galactosyliertes und/oder bis zu etwa 5 % fructosyliertes Süßungsglycosid hoher Intensität umfasst; oder
wobei die Zusammensetzung bis zu etwa 2 % galactosyliertes und/oder bis zu etwa 2 % fructosyliertes Süßungsglycosid hoher Intensität umfasst; oder
wobei die Zusammensetzung bis zu etwa 1,5 % galactosyliertes und/oder bis zu etwa 1,5 % fructosyliertes Süßungsglycosid hoher Intensität umfasst.

6. Zusammensetzung nach einem der vorstehenden Ansprüche, wobei das Süßungsglycosid hoher Intensität gleichzeitig mit der Synthese von Oligosacchariden galactosyliert und/oder fructosyliert worden ist.

7. Zusammensetzung nach Anspruch 6, wobei das Süßungsglycosid hoher Intensität Steviolglycoside umfasst, die durch Galactosylierung und/oder Fructosylierung mit bis zu etwa 3 Einheiten Galactose oder Fructose modifiziert sind; oder
wobei das Süßungsglycosid hoher Intensität Steviolglycoside umfasst, die durch Galactosylierung und/oder Fructosylierung mit bis zu etwa 4 Einheiten Galactose oder Fructose modifiziert sind; oder
wobei das Süßungsglycosid hoher Intensität Steviolglycoside umfasst, die durch Galactosylierung und/oder Fructosylierung mit mehr als 4 Einheiten Galactose oder Fructose modifiziert sind.

8. Zusammensetzung nach Anspruch 6, wobei das Süßungsglycosid hoher Intensität Mogroside umfasst, die durch Galactosylierung mit bis zu etwa 3 Einheiten Galactose modifiziert sind; oder
wobei das Süßungsglycosid hoher Intensität Mogroside umfasst, die durch Fructosylierung mit bis zu etwa 2 Einheiten Fructose modifiziert sind.

9. Zusammensetzung nach einem der Ansprüche 3 bis 7, wobei die Stevioside ein Gemisch von Steviosiden mit unterschiedlichen Modifikationen umfassen; und gegebenenfalls wobei die Zusammensetzung ein Gemisch von einem oder mehreren umfasst von: (i) Rebaudiosid A, Rebaudiosid F, Rebaudiosid C, Rubusosid oder Steviosid mit 1 Einheit Fructose; (ii) Steviosid mit 2 Einheiten Fructose oder Rebaudiosid A oder Rebaudiosid C mit 1 Einheit Fructose; und (iii) Steviosid mit 2 Einheiten Fructose oder Rebaudiosid A oder Rebaudiosid C mit 1 Einheit Fructose; oder
wobei die Zusammensetzung ein Gemisch von einem oder mehreren umfasst von: (i) Rebaudiosid A und Rebaudiosid C oder Steviosid mit 1 Einheit Galactose; (ii) Steviosid mit 2 Einheiten Galactose oder Rebaudiosid A oder Rebaudiosid C mit 1 Einheit Galactose; (iii) Steviosid mit 3 Einheiten Galactose oder Rebaudiosid A oder Rebaudiosid C mit 2 Einheiten Galactose; und (iv) Steviosid mit 4 Einheiten Galactose oder Rebaudiosid A oder Rebaudiosid C mit 2 Einheiten Galactose.

10. Zusammensetzung nach einem der Ansprüche 3 bis 6 und 8, wobei die Mogroside ein Gemisch von Mogrosiden mit unterschiedlichen Modifikationen umfassen; und wobei die Mogroside gegebenenfalls ein Gemisch von einem oder mehreren von Mogrosid II, Mogrosid III, Mogrosid IV, Mogrosid V oder Mogrosid VI umfassen; oder
wobei die Mogroside ein Gemisch von einem oder mehreren von umfassen von: (i) Mogrosid V; (ii) Mogrosid IV und (iii) Mogrosid III; oder
wobei die Mogroside ein Gemisch von einem oder mehreren von umfassen von: (i) Mogrosid III; (ii) Mogrosid IV; (iii) Mogrosid V; (iv) Mogrosid mit 1 Einheit Fructose; und (v) Mogrosid mit 2 Einheiten Fructose; oder
wobei die Mogroside ein Gemisch umfassen von: (i) Mogrosid IV; (ii) Mogrosid mit 1 Einheit Galactose; (iii) Mogrosid V mit 2 Einheiten Galactose; und (iv) Mogrosid V mit 3 Einheiten Galactose.

11. Verwendung der Zusammensetzung nach einem der vorstehenden Ansprüche zur Bereitstellung eines kalorienarmen oder kalorienfreien süßen Präbiotikums.

12. Verwendung der Zusammensetzung nach einem der Ansprüche 1 bis 10, wobei die Zusammensetzung in oder auf ein Lebensmittel, ein Nahrungsergänzungsmittel oder ein kalorienbeschränktes Mahlzeitersatzprodukt aufgenommen wird oder zur Aufnahme dient; und wobei die Zusammensetzung gegebenenfalls in einer granulatförmigen Form vorliegt und gegebenenfalls in einem Beutel oder einem Gefäß enthalten ist.

13. Verfahren zur Herstellung einer gesüßten präbiotischen Zusammensetzung, wobei das Verfahren umfasst:
Inkontaktbringen eines Süßungsglycosids hoher Intensität mit einem oder mehreren Enzymen, die wirksam sind, das Süßungsglycosid hoher Intensität in Gegenwart von Saccharose und/oder Lactose zu galactosylieren und/oder zu fructosylieren, um gleichzeitig verschiedene Oligosaccharide zu erzeugen; und gegebenenfalls wobei das Süßungsglycosid hoher Intensität ausgewählt ist aus einem oder mehreren der Folgenden: Steviolglycosiden oder Mogrosid oder Derivaten davon.

14. Verfahren nach Anspruch 13, wobei das Süßungsglycosid hoher Intensität unter Verwendung eines oder mehrerer Enzyme galactosyliert und/oder fructosyliert wird, die ausgewählt sind aus: Carbohydrasegemischen, erhalten aus Aspergillus sp., und β-Galactosidasen; und gegebenenfalls wobei das synthetisierte Oligosaccharid eines oder mehrere der Folgenden ist: Galactooligosaccharide (GOS) oder Fructooligosaccharide (FOS).

15. Verfahren nach einem der Ansprüche 13 oder 14, wobei die gesüßte präbiotische Zusammensetzung bis zu etwa 5 % galactosyliertes und/oder bis zu etwa 5 % fructosyliertes Süßungsglycosid hoher Intensität umfasst.

## Revendications

1. Composition prébiotique obtenue en modifiant enzymatiquement un glycoside édulcorant de haute intensité par galactosylation et/ou fructosylation en présence de saccharose ou de lactose, et en obtenant le glycoside édulcorant de haute intensité modifié enzymatiquement et un oligosaccharide différent, dans laquelle la composition prébiotique comprend :
(i) le glycoside édulcorant de haute intensité modifié enzymatiquement ; et
(ii) l'oligosaccharide.

2. Composition selon la revendication 1, dans laquelle le glycoside édulcorant de haute intensité est choisi parmi un ou plusieurs des suivants : Mogroside ou glycosides de Stéviol, ou leurs dérivés.

3. Composition selon la revendication 2, dans laquelle le glycoside de Stéviol comprend le Rébaudioside A ou le Mogroside comprend le Mogroside V.

4. Composition selon la revendication 1, dans laquelle l'oligosaccharide est un ou plusieurs des suivants : galactooligosaccharides (GOS) ou fructo-oligosaccharides (FOS).

5. Composition selon l'une quelconque des revendications précédentes, dans laquelle la composition comprend jusqu'à environ 5 % de glycoside édulcorant de haute intensité galactosylé et/ou jusqu'à environ 5 % de glycoside édulcorant de haute intensité fructosylé ; ou dans laquelle la composition comprend jusqu'à environ 2 % de glycoside édulcorant de haute intensité galactosylé et/ou jusqu'à environ 2 % de glycoside édulcorant de haute intensité fructosylé ; ou
dans laquelle la composition comprend jusqu'à environ 1,5 % de glycoside édulcorant de haute intensité galactosylé et/ou jusqu'à environ 1,5 % de glycoside édulcorant de haute intensité fructosylé.

6. Composition selon l'une quelconque des revendications précédentes, dans laquelle le glycoside édulcorant de haute intensité a été galactosylé et/ou fructosylé simultanément avec la synthèse d'oligosaccharides.

7. Composition selon la revendication 6, dans laquelle le glycoside édulcorant de haute intensité comprend des glycosides de stéviol qui sont modifiés par jusqu'à environ 3 unités de galactose ou fructose par galactosylation et/ou fructosylation ; ou
dans laquelle le glycoside édulcorant de haute intensité comprend des glycosides de stéviol qui sont modifiés par jusqu'à environ 4 unités de galactose ou fructose par galactosylation et/ou fructosylation ; ou.
dans laquelle le glycoside édulcorant de haute intensité comprend des glycosides de stéviol qui sont modifiés par plus de 4 unités de galactose ou fructose par galactosylation et/ou fructosylation.

8. Composition selon la revendication 6, dans laquelle le glycoside édulcorant de haute intensité comprend des mogrosides qui sont modifiés par jusqu'à environ 3 unités de galactose par galactosylation ; ou
dans laquelle le glycoside édulcorant de haute intensité comprend des mogrosides qui sont modifiés par jusqu'à environ 2 unités de fructose par fructosylation.

9. Composition selon l'une quelconque des revendications 3 à 7, dans laquelle les stéviosides comprennent un mélange de stévisiodes ayant différentes modifications ; et, éventuellement, dans laquelle la composition comprend un mélange de un ou plusieurs parmi :
(i) rébaudioside A, rébaudioside F, rébaudioside C, rubusoside ou stévioside ayant 1 unité de fructose ; (ii) stévioside ayant 2 unités de fructose ou rébaudioside A ou rébaudioside C ayant 1 unité de fructose ; et (iii) stévioside ayant 2 unités de fructose ou rébaudioside A ou rébaudioside C ayant 1 unité de fructose ; ou
dans laquelle la composition comprend un mélange d'un ou plusieurs parmi : (i) rébaudioside A et rébaudioside C ou stévioside ayant 1 unité de galactose ; (ii) stévioside ayant 2 unités de galactose ou rébaudioside A ou rébaudioside C ayant 1 unité de galactose ; (iii) stévioside ayant 3 unités de galactose ou rébaudioside A ou rébaudioside C ayant 2 unités de galactose ; et (iv) stévioside ayant 4 unités de galactose ou rébaudioside A ou rébaudioside C ayant 2 unités de galactose.

10. Composition selon l'une quelconque des revendications 3 à 6 et 8, dans laquelle les mogrosides comprennent un mélange de mogrosides ayant différentes modifications ; et éventuellement dans laquelle les mogrosides comprennent un mélange d'un ou plusieurs parmi le mogroside II, le mogroside III, le mogroside IV, le mogroside V ou le mogroside VI ; ou
dans laquelle les mogrosides comprennent un mélange d'un ou plusieurs parmi : (i) mogroside V ; (ii) mogroside IV ; et (iii) mogroside III ; ou
dans laquelle les mogrosides comprennent un mélange d'un ou plusieurs parmi : (i) mogroside III ; (ii) mogroside IV ; (iii) mogroside V ; (iv) mogroside ayant 1 unité de fructose ; et (v) mogroside ayant 2 unités de fructose ; ou
dans laquelle les mogrosides comprennent un mélange de :
(i) mogroside IV ; (ii) mogroside ayant 1 unité de galactose ; (iii) mogroside V ayant 2 unités de galactose ; et (iv) mogroside V ayant 3 unités de galactose.

11. Utilisation de la composition selon l'une quelconque des revendications précédentes pour fournir un prébiotique sucré à faible teneur en calories ou sans calories.

12. Utilisation de la composition selon l'une quelconque des revendications 1 à 10, dans laquelle la composition est incorporée, ou est destinée à être incorporée, dans ou sur, un produit alimentaire, un supplément alimentaire ou un produit de remplacement de repas à teneur réduite en calories ; et facultativement dans laquelle la composition est sous forme granulaire, et facultativement, incluse dans un sachet ou un pot.

13. Procédé de production d'une composition prébiotique édulcorée, le procédé comprenant :
mise en contact d'un glycoside édulcorant de haute intensité avec une ou plusieurs enzymes efficaces pour galactosyler et/ou fructosyler le glycoside édulcorant de haute intensité en présence de saccharose et/ou de lactose de manière à produire simultanément des oligosaccharides différents ; et facultativement, dans lequel le glycoside édulcorant de haute intensité est choisi parmi un ou plusieurs des suivants : Mogroside ou glycosides de Stéviol, ou leurs dérivés.

14. Procédé selon la revendication 13, dans lequel le glycoside édulcorant de haute intensité est galactosylé et/ou fructosylé en utilisant une ou plusieurs enzymes choisies parmi : des mélanges de carbohydrase obtenus à partir d'Aspergillus sp. et des β-galactosidases ; et éventuellement dans lequel l'oligosaccharide synthétisé est un ou plusieurs des suivants : galactooligosaccharides (GOS) ou fructooligosaccharides (FOS).

15. Procédé selon l'une quelconque des revendications 13 ou 14, dans lequel la composition prébiotique édulcorée comprend jusqu'à environ 5 % de glycoside édulcorant de haute intensité galactosylé et/ou jusqu'à environ 5 % de glycoside édulcorant de haute intensité fructosylé.
